# EUROPEAN PATENT APPLICATION

(11) **EP 0 983 764 A1**
(43) Date of publication of application: **08.03.2000**
(21) Application number: 98911142.2
(22) Date of filing: 31.03.1998
(51) Int. Cl.: A61K 31/165

(54) **PREVENTIVES FOR ISCHEMIC HEART DISEASES**

(30) Priority: 01.04.1997 JP 8265097
(71) Applicant: TAISHO PHARMACEUTICAL CO. LTD, Tokyo 170-8633 (JP)
(72) Inventor: ASAMI, Yumiko, Taisho Pharmaceutical Co., Ltd, Toshima-ku, Tokyo 170-8633 (JP); AKIYOSHI,Kazuhiko. Taisho Pharmaceutical Co., Ltd, Toshima-ku, Tokyo 170-8633 (JP); YAMAGISHI, Izumi, Taisho Pharmaceutical Co., Ltd, Toshima-ku, Tokyo 170-8633 (JP); TOMOIKE, Hideki, Taisho Pharmaceutical Co., Ltd, Toshima-ku, Tokyo 170-8633 (JP)
(74) Representative: Albrecht, Thomas, Dr.
(86) International application number: JP9801487
(87) International publication number: WO9843627

(57) **Abstract**

Preventives for post-complication and ischemic heart diseases characterized by containing as the active ingredient a compound represented by formula (I), which inhibits arterial plaque (intimal thickening) diabrosis in patients with arteriosclerosis to thereby prevent subsequent various ischemic diseases.

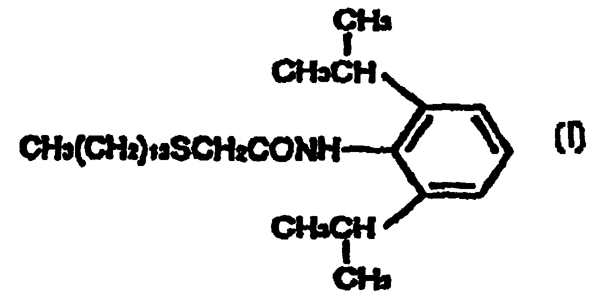

## Description

### TECHNICAL FIELD

This invention relates to prophylaxis of various secondary ischemic diseases in patients suffering from arteriosclerosis by inhibiting the disruption of the arterial wall plaque (intimal thickening) of said patients.

### BACKGROUND ART

Arteriosclerosis is one of the major basic lesions of ischemic diseases. There is seen a difference in the components of the lesion depending upon the arterial sites at the initial stage of arteriosclerosis, while diffuse intimal thickening caused by smooth muscle cells in coronary artery may develop even in teen-agers. The thickening gradually progresses with age and eventually grows to the plaque which is an atheromatous sclerotic lesion raised within the arterial lumen and occupies a considerable area. The American Heart Association classified the plaques into six types based upon the changes in the plaque components and determined that lipid accumulation is the first and major mechanism of the progression of plaque formation (Stary, H.C., Circulation, 92, pp. 1355-1374 (1995)).

It has been recently elucidated that the onset of acute coronary syndromes such as unstable angina pectoris or acute myocardial infarction closely relates to the disruption of plaque caused by instabilization of components in plaque rather than severe stenosis of the blood vessel (Falk, E. et al., Circulation, 92, pp. 657-671 (1995)).

### DISCLOSURE OF INVENTION

It is said that the plaque is classified depending upon the components thereof into one type which may be hardly disrupted (stable plaque) and another type which may be easily disrupted (unstable plaque). The stable plaque is composed mainly of extracellular matrix components such as fibers and cellular components such as smooth muscle cells, and has a considerably thick fibrous cap which covers small-scale lipid depositions, if any, in the deep intima.

On the other hand, the unstable plaque is the plaque which is composed mainly of lipid components such as cholesteryl esters, in which there is seen infiltration of macrophages or inflammatory cells, and which has a highly thinned and weakened fibrous cap covering the lumen side of the blood vessel (Falk, E. et al., Circulation, 92, pp. 657-671 (1995)). The plaque will be injured up to the deep part thereof by the disruption of the unstable plaque, whereby atheromatous gruel is contacted with blood to induce thrombus and thrombus formation intensively progresses in the center of atheroma, which leads to occlusion of the blood vessel and subsequent evolution of heart events (Fuster, V. et al., N. Eng. J. Med., 326, pp. 242-250 and 310-318 (1992)).

Not only in the coronary artery, a part of the thrombus caused by the plaque disruption of atherosclerosis is transferred as the embolus to the upper fine blood vessel to form thrombus therein, which is closely related with the onset of ischemic diseases such as transient ischemic attack, cerebral infarction or peripheral arterial thrombus.

In view of the above problems, the present inventors have conducted a screening of a rabbit or hamster model for arteriosclerosis, and as a result, have found out an effective compound, upon which the invention has been completed.

More specifically, the invention is directed to an agent for preventing ischemic heart diseases based upon a stabilizing effect on the plaque, which comprises as an active ingredient a compound represented by the formula

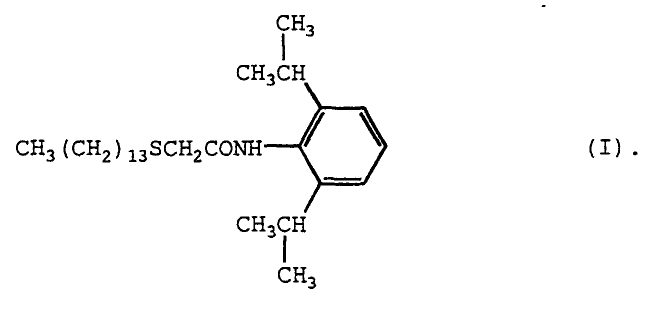

The compound represented by the formula (I) (hereinafter referred to as the compound of the invention) could inhibit the deposition of lipid in the arterial wall even at such a low dose as not to lower a blood cholesterol level in a hamster model for arteriosclerosis. The lipid deposition in this model is said to be the initial lesion in which the monocyte-derived macrophages may store up cholesteryl esters to form foam cells which are then deposited in the blood vessel (Mark C. Kowala, et al., Arteriosclerosis and Thrombosis, 13, pp. 435-444 (1993)).

It is considered that the ACAT inhibiting action by the compound of the invention would most greatly contribute to the action of the compound of the invention to inhibit cholesterol accumulation by macrophages, while, at the preceding steps of adhesion of monocytes onto the arterial wall and macrophage formation, it is considered that there is another mechanism of action such as inhibition of leukotriene synthesis and promotion of the production of prostacyclins. It is apparent from this action that the compound of the invention may inhibit the lipid deposition onto the blood vessel wall from its early phase, which is the most significant and first mechanism of action for the progression of plaque.

In a model for treating arteriosclerosis in a cholesterol-loading rabbit as used by the present inventors, the common carotid arteries are pathologically in the growth phase of atheroma wherein intimal thickening is progressing with the unstable plaque enriched with lipid components mainly composed of macrophages. The compound of the invention inhibited the growth of plaque at this phase and showed an inhibiting activity against intimal thickening.

The aortic arch at this stage is in the phase of the complete plaque wherein the progression of intimal thickening has already come to an end. Pathologically, macrophages and numerous smooth muscle cells are intermingled, intracellular and extracellular lipid components are rich, and a fibrous cap of the incomplete plaque is constructed.

Although the compound of the invention exhibited no action of regressing a degree of intimal thickening, it reduced the lipid components in plaque, and contrarily, promoted the production of fibrous components and accelerated the construction of the fibrous cap composed of smooth muscle cells and elastic fibers of elastin in the lumen side of the blood vessel.

In short, the compound of the invention promoted the stabilization of plaque. The development of these actions has been confirmed at such a dose that the compound of the invention would not lower a blood cholesterol level.

The effect on multiple arteriosclerotic lesions has also been studied by continuously administering the compound of the invention to a hereditary hyperlipemic WHHL rabbit, which is the animal model for familial hyperlipemia which may develop juvenile ischemic heart diseases, over a prolonged period of time from the young age period before the development of the lesion up to the old age period of the latter multiple lesions. The aortic lesion of an old WHHL rabbit of 20 months at autopsy formed the multiple lesions wherein collagen and elastin were intermingled with smooth muscle cells and macrophages, and there were observed the deposition of numerous cholesterol crystals and calcium, the injured intima and the weakened media.

The compound of the invention showed the actions of lowering a cholesterol content in the arterial wall, and further, modifying the components of lesion sites to reinforce and stabilize the construction of the blood vessel. Thus, it inhibited the deposition of cholesterol crystals in plaque.

It further promoted the production of the intimal elastin, inhibited the production of collagen and especially increased a ratio of elastin/collagen in the plaque cortex part. This is considered to represent that it induces intimal fibrous components to a normal type for constructing the blood vessel for the medium.

Moreover, the compound of the invention exhibited the actions of promoting the production of the medium elastin and compacting endothelial cells. Since the model is the old lesion developing the weakened media and injured endothelia, there is a possibility that the compound of the invention may exert an action of improving the whole vascular lesions by reinforcing the construction of the blood vessel.

These results show that the compound of the invention exerted the action directly onto multiple vascular lesions at such a dose as not to cause any change in a blood cholesterol level.

It is considered that the ACAT inhibiting activity of the compound of the invention is most deeply concerned in reducing lipid components in plaque. In reference to the promoting action on the production of fibrous components, there is another mechanism of action of the compound of the invention on smooth muscle cells.

Since elastin is predominant in smooth muscle cells of a contraction type in the normal medium, it is considered that the compound of the invention may induce smooth muscle cells of a contraction type into the intima by either direct or indirect action and may promote the organic reinforcement of the blood vessel wall, which results in the improvement in the whole functions of the blood vessel.

The compound of the invention is a well-known compound as disclosed in Japanese Patent Kokai No. 40898/1994 (corresponding to U.S. Patent No. 5,475,130).

### BEST MODE FOR CARRYING OUT THE INVENTION

A dose of the compound which is an active ingredient in the invention may vary depending upon the conditions. A daily dose for adults is usually 0.5-120 mg/human in the case of oral administration and it may be administered once per day or in several divided doses per day.

The preventive agent of the invention may be applied after prepared in a solid form such as tablets, pills, capsules, granules, dry syrups, etc.

In preparing a solid preparation, various additives may be used, for example, excipients, disintegrators, binders, lubricants or coating bases, and an agitation granulation method, a fluidized bed granulation method or a fracture granulation method can be employed.

The excipients may include mannitol, xylitol, sorbitol, glucose, sucrose, lactose, crystalline cellulose, crystalline cellulose·carboxymethylcellulose sodium, calcium hydrogenphosphate, wheat starch, rice starch, corn starch, potato starch, carboxymethylstarch sodium, dextrin, a fatty acid triglyceride, etc.

The disintegrators may include a hydroxypropyl cellulose with a low degree of substitution, carboxymethylcellulose, carboxymethyl-cellulose sodium, starch, crystalline cellulose, hydroxypropylstarch, etc.

The binders may include, for example, methylcellulose, hydroxypropylcellulose, hydroxypropylmethylcellulose, polyvinyl pyrrolidone, gelatin, gum arabic, ethylcellulose, polyvinyl alcohol, pullulan, agar, tragacanth, etc.

The lubricants may include stearic acid, magnesium stearate, calcium stearate, talc, a hardened oil, a sucrose fatty acid ester, etc.

The antioxidants may include dibutylhydroxytoluene (BHT), pyrogallic acid propyl ester, butylhydroxyanisole (BHA), α-tocopherol, citric acid, etc.

The coating agents may include hydroxypropylmethylcellulose, hydroxypropylcellulose, methylcellulose, ethylcellulose, hydroxypropylmethylcellulose phthalate, hydroxypropylmethylcellulose acetate succinate, carboxymethylethylcellulose, etc.

The coloring agents may include a tar color, titanium oxide, etc.

The corrigents may include citric acid, adipic acid, ascorbic acid, menthol, etc.

The surfactants may include polyoxyethylene hardened castor oil, glycerol monostearate, sorbitan monostearate, sorbitan monopalmitate, polysorbates, sodium lauryl sulfate, Macrogols, a sucrose fatty acid ester, etc.

The plasticizers may include triethyl citrate, triacetin, cetanol, etc.

The agent for preventing ischemic heart diseases of the invention which is based upon the plaque-stabilizing activity may be applied orally or parenterally in any other form suitable for each administration. Example of such forms may be injections, solutions, emulsions, suppositories, etc.

### INDUSTRIAL APPLICABILITY

The compound of the invention can inhibit the adhesion, infiltration and foaming of macrophages which are initial lesions in instabilization of plaque, and inhibit the intimal thickening during the growth phase of plaque.

The compound of the invention further eliminates lipids from the components in plaque to promote fibrogenesis, which leads to stabilization of plaque, and plaque disruption can be prevented by such plaque stabilizing activities. Thus, it can provide a useful drug which can prevent the onset of ischemic heart diseases caused by the subsequent thrombotic occlusion.

The invention will be more fully explained by way of the following Test Examples and Examples. Test Example 1 [Antiarteriosclerotic activity in a model for the treatment of arteriosclerosis in high fat-loading hamsters]

### (Test Procedure)

As test animals were used a group of 5 male golden hamsters of 6 weeks (available from Nihon SLC K.K.) Hamsters were fed with the following high fat diet for 10 weeks to prepare a model for arteriosclerosis.

During this period, the compound of the invention was continuously administered to a medicated group, simultaneously with feeding.

After blood collection, the aortic arch was excised and the adventitia was completely stripped off under microscopic observation. The tissue after fixed with 10% neutral buffered formalin was fat-stained with an Oil Red O solution and enclosed in a glycerol-gelatin solution on a slide glass to prepare a tissue preparation.

An area of the fat-stained site (µm²) and an area of the artery (mm²) were measured by means of an image analysis system (Adobe Photoshop, Unitimage for the Apple Macintosh) and a ratio of both values was calculated to determine a degree of lipid deposition on the arterial wall.

A total blood cholesterol level was measured according to an enzyme method (Autosera CHO-2, Daiichi Kagaku Yakuhin K.K.) using an autoanalyzer (Hitachi 7150 type).

The drug was given in admixture with a high fat diet at the concentrations of 0.003% and of 0.03%, and a dose was calculated from the amount of feed ingested and the body weight.

Composition of a high fat diet: A feed for rats MF (available from Oriental Kobo K.K.) to which 10% coconut oil and 0.05% cholesterol were supplemented. (Results)

The results are shown in Table 1.

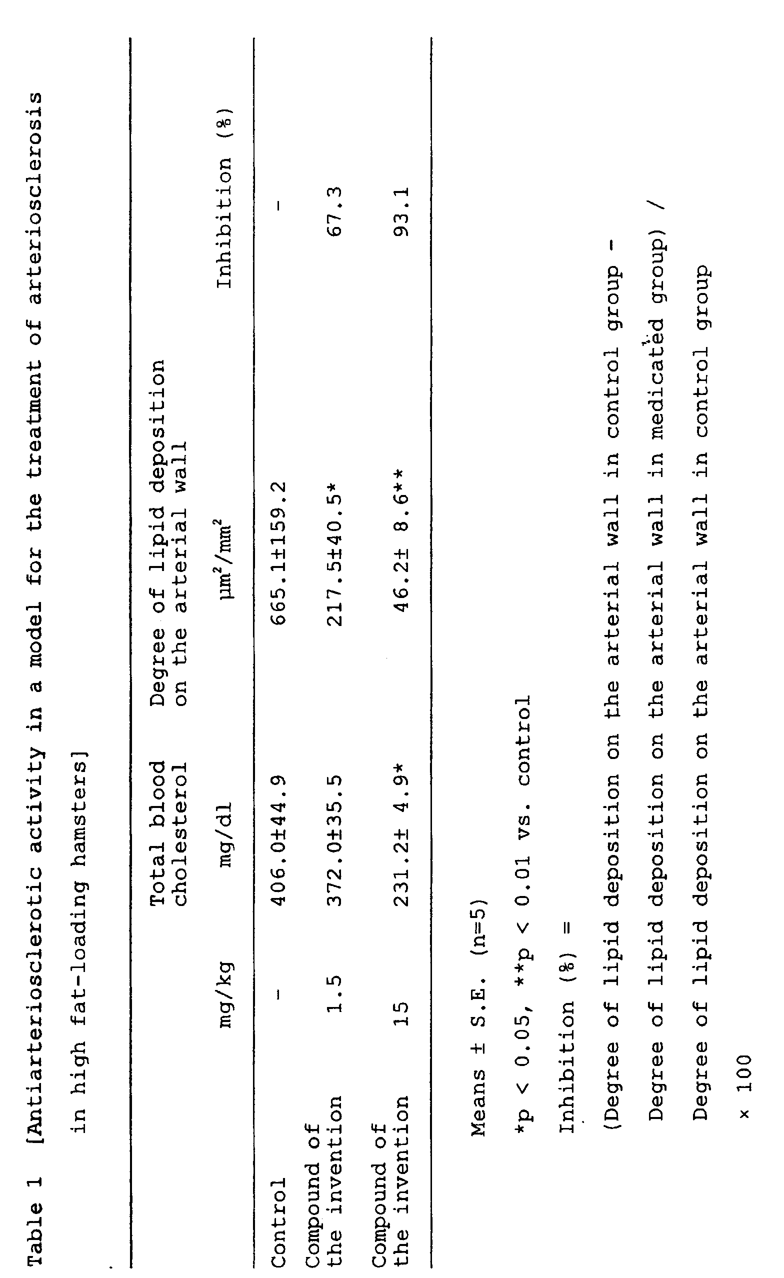

### Test Example 2 [Antiarteriosclerotic activity in a model for the treatment of arteriosclerosis in cholesterol-loading rabbits]

### (Test Procedure)

As test animals were used a group of 10 rabbits previously fed with a 1% cholesterol-enriched feed to develop atherosclerosis (of 5 months, NZW strain male; available from Kitayama RABES K.K.)

A group of rabbits with arteriosclerosis were fed with a normal feed for rabbits RC-4 (available from Oriental Kobo K-K.) for 3 months and then adopted as a control group, while the compound of the invention was orally administered to a medicated group at 3 mg/kg once per day during this period.

After blood collection, the common carotid artery and the aorta were excised and a certain site thereof was fixed with 10% neutral buffered formalin to prepare a slice of the arterial tissue. Elastin elastic fiber-stained preparations were prepared by Elastic Van Gieson (EVG) staining, fat-stained preparations were prepared by Oil Red O staining and smooth muscle-stained preparations were prepared by anti-human α-actin antibody 1A4 staining.

A degree of intimal elastin fibrogenesis and a degree of medium lipid deposition were assessed in terms of 5 scores on elastin-positve and lipid staining-positive intensities (Grade; 0: No changes, 1: Minimum, 2: Mild, 3: Moderate, and 4: Severe). Areas of the intima (I) and of the medium (M) were measured by means of an image analysis system (Adobe Photoshop, Unitimage for the Apple Macintosh) and a ratio of both values (I/M) was calculated.

An elastin density of the fibrous cap in the intimal surface was calculated in terms of a ratio of the elastin-positive area to a given area of the intimal surface. A ratio of the α-actin-positive area to the intimal surface area at a similar site was defined as a smooth muscle density. Cholesterol was extracted from a portion of the excised aorta with a solution of chloroform : methanol (2:1) and was assayed according to an enzyme method (Cholesterol CII-Test WAKO, Free Cholesterol C-TEST WAKO, Wako Pure Chemical Industries, Ltd.)

A total blood cholesterol level was measured according to an enzyme method (Autosera CHO-2, Daiichi Kagaku Yakuhin K.K.) using an autoanalyzer (Hitachi 7150 type).

### (Results)

The results are shown in Tables 2, 3, 4 and 5.

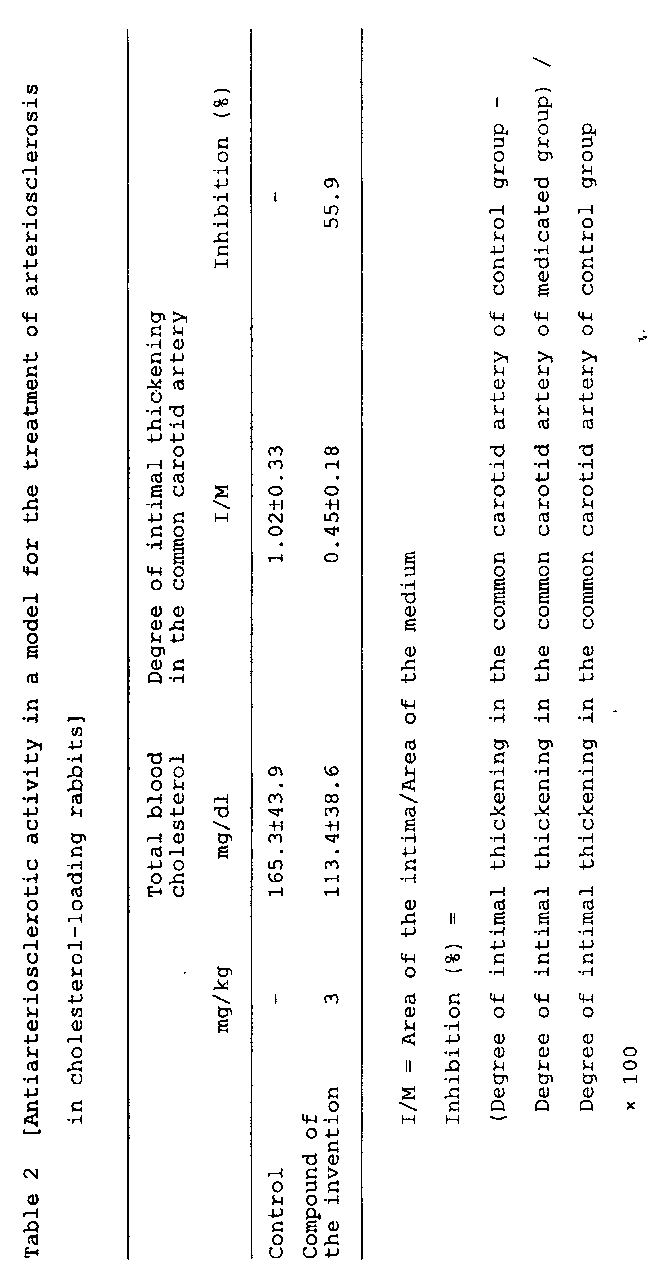

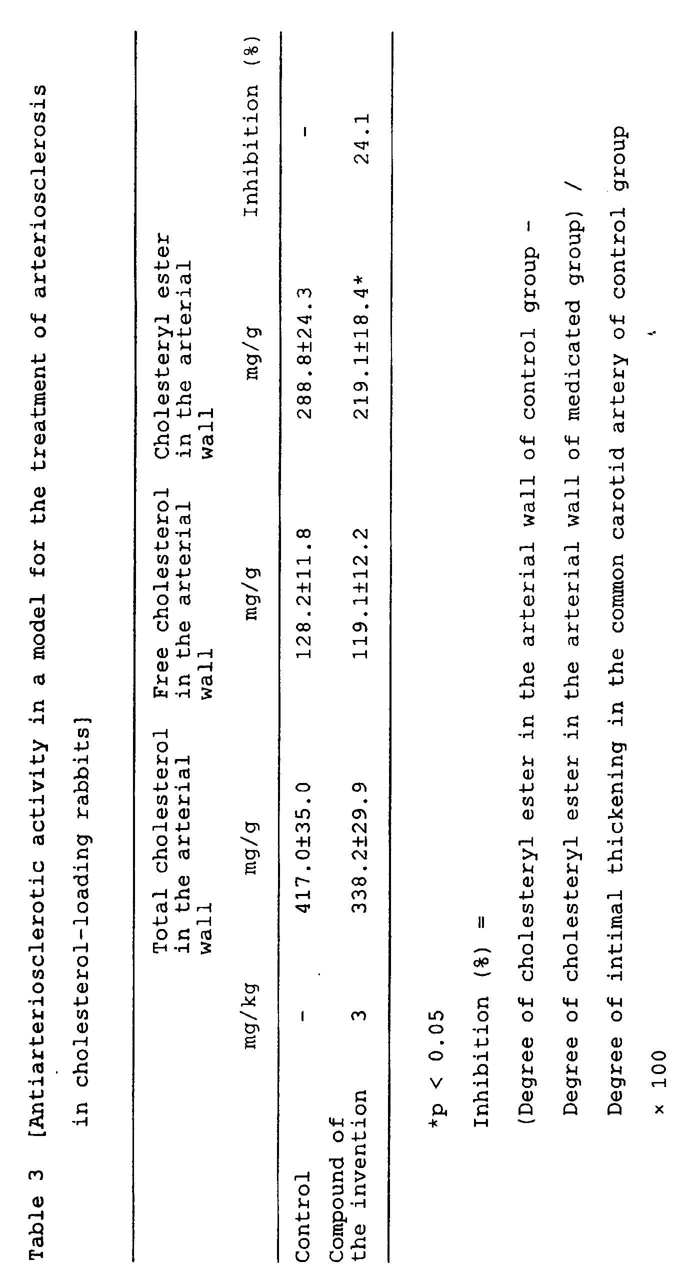

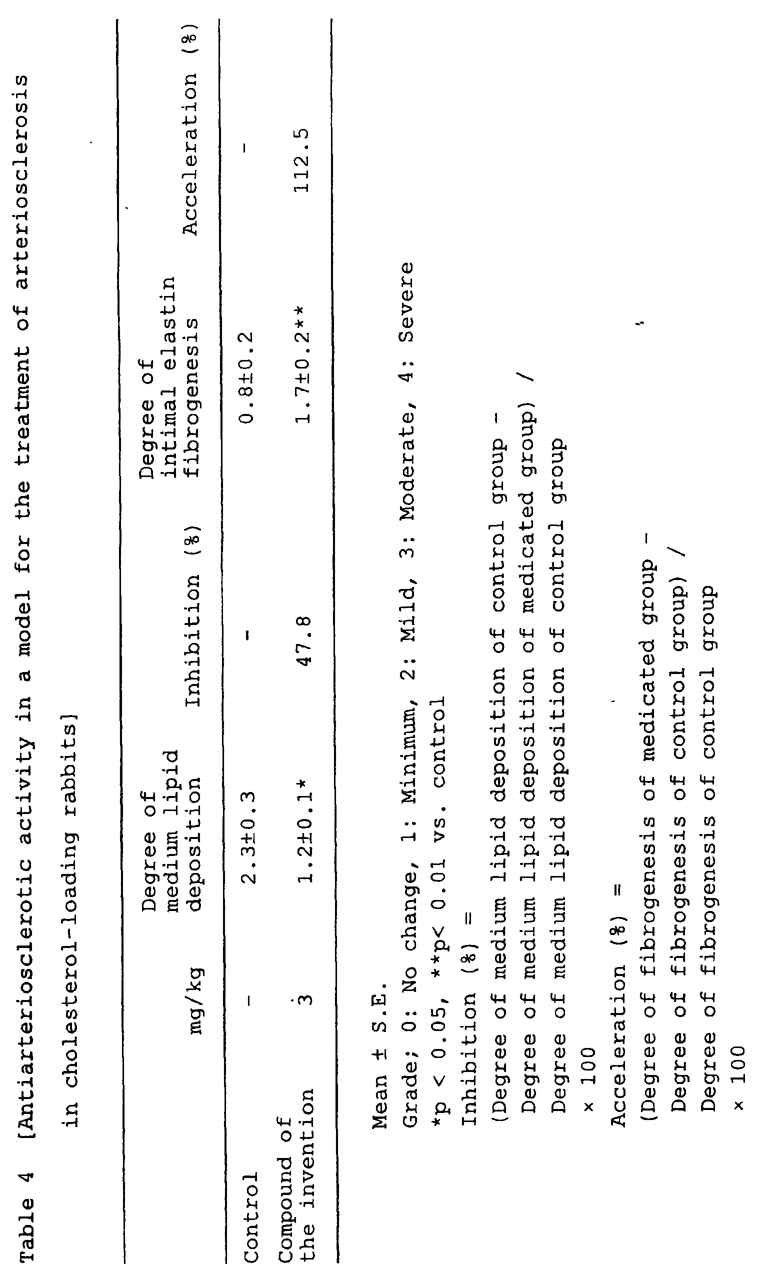

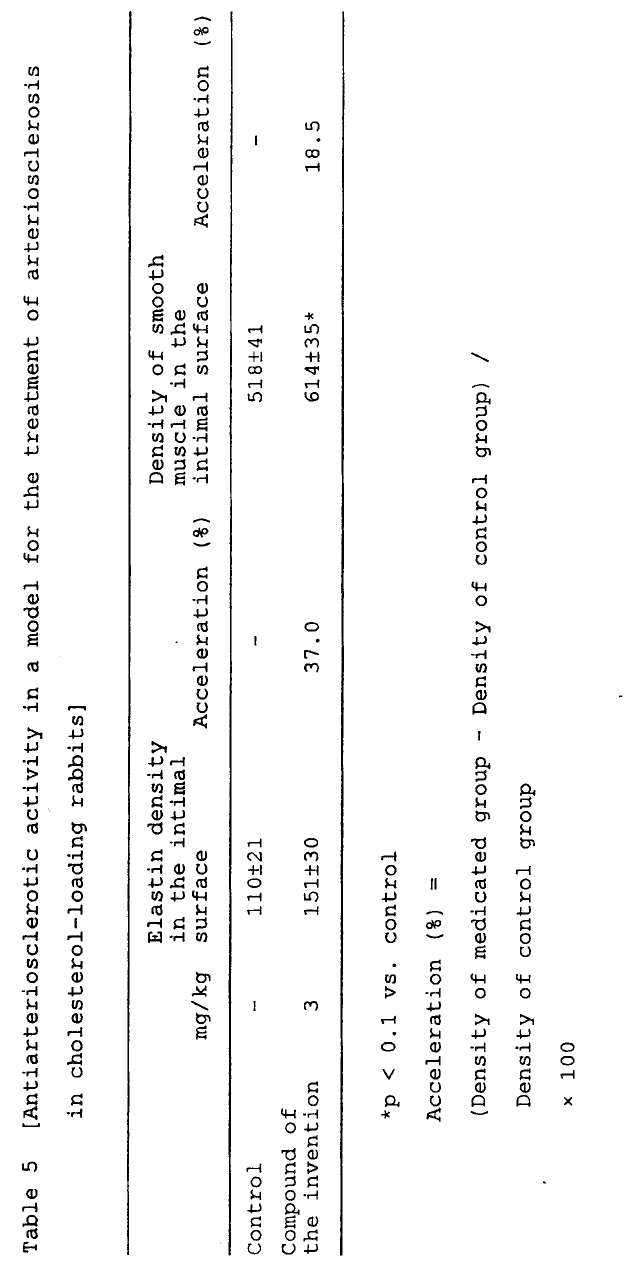

### Test Example 3 [Antiarterioscierotic activity in hereditary hyperlipemic WHHL rabbits]

### (Test Procedure)

The compound of the invention was continuously administered to rabbits of 2 months with hereditary hyperlipemia (WHHL rabbits, available from Kitayama RABES K.K.) over 18 months and then the rabbits were autopsied.

A portion of the aorta was fixed with 10% neutral buffered formalin and then embedded in paraffin or freeze-embedded to prepare a slice of the arterial tissue. The slice was stained with Hematoxylin and Eosin (HE), Elastic Van Gieson (EVG), Azan-Mallory, and Oil Red O. A positive rate was assessed under microscopic observation in terms of 5 scores (Grade; 0: No changes, 1: Minimum, 2: Mild, 3: Moderate, and 4: Severe) to measure changes in components in the arteriosclerotic lesion. For another portion of the aorta, cholesterol was extracted with a solution of chloroform methanol (2:1) and a total cholesterol level in the arterial wall was assayed according to an enzyme method (Cholesterol CII-Test WAKO, Wako Pure Chemical Industries, Ltd.)

A total blood cholesterol level value at the autopsy was measured according to an enzyme method (Autosera CHO-2, Daiichi Kagaku Yakuhin K.K.) using an autoanalyzer (Hitachi 7150 type).

The drug was mixed into a feed for rabbits RC-4 (Oriental Kobo K.K.) at the concentrations of 0.005% and of 0.05%, and the resulting feed was given daily between 9:00 a.m. and 11:30 a.m. while the feed was restricted to 20 g/kg of body weight.

### (Results)

The results are shown in Tables 6 and 7.

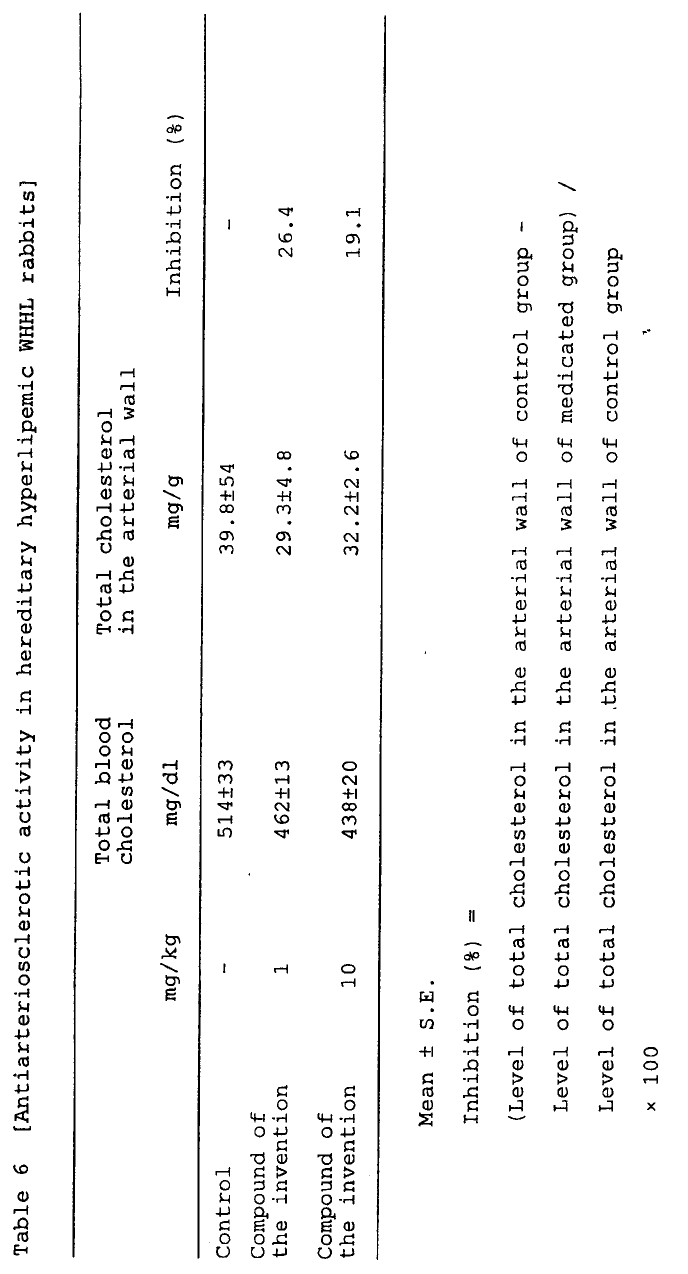

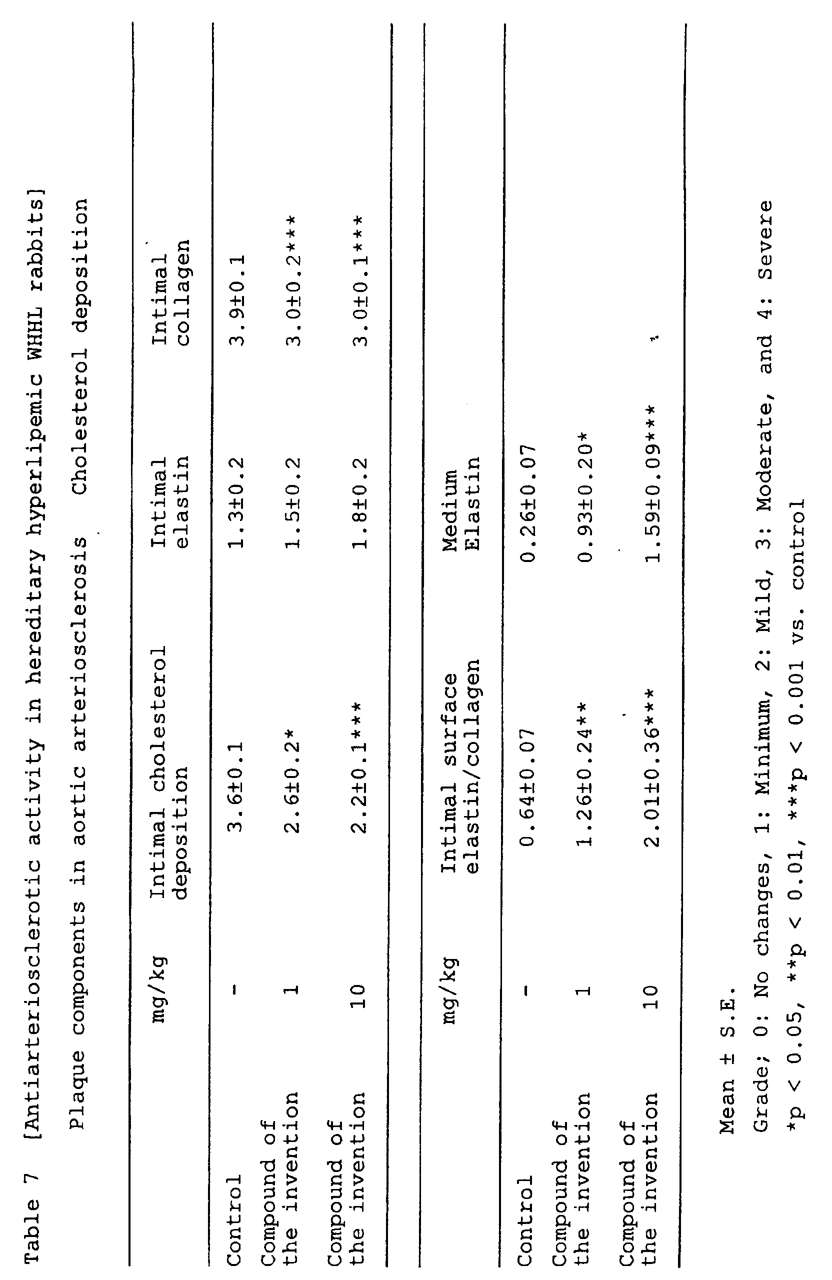

### Example 1

In 945 g of a middle chain fatty acid triglyceride and 5 g of Polysorbate 80 was dissolved 50 g of the compound of the invention to prepare a solution for encapsulation.

### Example 2

In 895 g of oleic acid and 5 g of Polysorbate 80 was dissolved 100 g of the compound of the invention to prepare a solution for encapsulation.

### Example 3

In 985 g of soybean oil and 5 g of Polysorbate 80 was dissolved 10 g of the compound of the invention to prepare a solution for encapsulation.

### Example 4

In 945 g of a middle chain fatty acid triglyceride and 5 g of the Polysorbate was dissolved 50 g of the compound of the invention. 10 g of the resulting solution was adsorbed into a premix of 20 g of calcium silicate and 40 g of calcium hydrogenphosphate, and then 29 g of mannitol was added to prepare a powdery mixture. 1 g of magnesium stearate was further added to prepare powders.

### Example 5

In 945 g of a middle chain fatty acid triglyceride and 5 g of the Polysorbate was dissolved 50 g of the compound of the invention. 10 g of the resulting solution was adsorbed into a premix of 20 g of calcium silicate and 40 g of calcium hydrogenphosphate, and then 24 g of lactose was added to prepare a powdery mixture. 1 g of magnesium stearate and 5 g of talc were further added, and the mixture was uniformly admixed and then filled into capsules to prepare capsules.

### Example 6

In 945 g of a middle chain fatty acid triglyceride and 5 g of the Polysorbate was dissolved 50 g of the compound of the invention. 10 g of the resulting solution was adsorbed into a premix of 20 g of calcium silicate and 40 g of calcium hydrogenphosphate, and then 24 g of crystalline cellulose and 10 g of carmellose calcium were added to prepare a powdery mixture. 1 g of magnesium stearate and 5 g of talc were further added, and the mixture was uniformly admixed and then tableted by means of a tableting machine to prepare tablets.

### Example 7

### Formulation (per one capsule)

| | |
|---|---|
| The compound of the invention | 30 mg |
| Oleic acid | 268.65 mg |
| Polysorbate 80 | 1.35 mg |
| Total | 300 mg |

In the oleic acid and the Polysorbate 80 was dissolved the compound of the invention to prepare a solution for encapsulation.

## Claims

1. An agent for preventing ischemic heart diseases upon a plaque-stabilizing activity, which contains as an active ingredient a compound represented by the formula
